Europäisches Patentamt

**European Patent Office**

**Office européen des brevets**

(11) Publication number: **0 265 002 B1**

# EUROPEAN PATENT SPECIFICATION

(45) Date of publication of patent specification: **03.07.91**  (51) Int. Cl.⁵: **A61L 2/18, B05B 9/04**

(21) Application number: **87201929.4**

(22) Date of filing: **08.10.87**

(54) Device for transferring antiseptic solution.

(30) Priority: **13.10.86 JP 242323/86**

(43) Date of publication of application:
**27.04.88 Bulletin 88/17**

(45) Publication of the grant of the patent:
**03.07.91 Bulletin 91/27**

(84) Designated Contracting States:
**CH DE FR GB LI NL SE**

(56) References cited:
**WO-A-79/01074**
**US-A- 2 668 637**

(73) Proprietor: **Shikoku Kakoki Co., Ltd.**
**10-1, Aza-Nishinokawa Tarohachizu Kitajima-cho**
**Itano-gun Tokushima(JP)**

(72) Inventor: **Hayashi, Kojiro c/o Shikoku Co. Ltd.**
**10-1, Aza-Nishinokawa Tarohachizu**
**Kitajima-cho Itano-gun Tokushima(JP)**

(74) Representative: **Noz, Franciscus Xaverius, Ir. et al**
**Algemeen Octrooibureau P.O. Box 645**
**NL-5600 AP Eindhoven(NL)**

## Description

The present invention relates to a device for spraying hydrogen peroxide water-solution on food packaging containers to be sterilized comprising a tank for containing the hydrogen peroxide water-solution and a tube for feeding the water-solution from the tank to a spray nozzle.

From WO/01074 there is known a device for sterilization of containers wherein hydrogen peroxide is supplied to a spray nozzle from a tank by means of a nebulizing chamber. In said nebulizing chamber there has been mounted a tranducer for producing vibrational energy in order to nebulize the liquid. Pressurized air is fed to the nebulizing chamber in order to carry over the automized particles of hydrogen peroxide to the nozzle.

By the applicant devices are experimented which comprises a tube, and a gear pump, diaphragm pumps or the like disposed at an intermediate portion of the tube. These pumps also encounter difficulty in transferring the antiseptic solution at a constant rate since the efficiency of the pump varies with the amount of oxygen bubbles formed by decomposition of the hydrogen oxide. Furthermore, the negative pressure produced in the vicinity of the suction port of the pump permits formation of a larger amount of bubbles, consequently lowering the pump efficiency. In the case of the diaphragm pump which requires a chech valve, oxygen is liable to remain in the form of bubbles at the location of the check valve. The pump is then likely to forward bubbles only.

The main object of the present invention is to provide a device for transferring a hydrogen peroxide water-solution free of the foregoing problems.

According the invention this can be obtained by a device as described in claim 1.

In using the device according this invention the hydrogen peroxide water-solution is transferred from the tank to the nozzle through the tube by the tube pump which successively collapses the tube from portion to portion in a direction from the tank toward the nozzle, so that even when the solution contains bubbles, the solution can be sent forward irrespective of the bubbles. Since the operation of the tube pump does not produce a large amount of bubbles within the tube while transporting the solution, the solution can be transferred at a constant flow rate with good stability unlike the test wherein the above mentioned pump was used. The solution does not evolve a large quantity of bubbles within the tube presumably because the suction pressure for drawing the solution into the tube is produced by the tube when it acts to elastically restort itself to the original shape, and therefore vary smoothly. Moreover, no bubbles remain in the tube pump since the feeder does not require any check valve unlike the aforementioned pump.

It is noted that from Ullman Enzyklopädi vol.3 page 169/vol.5 page 887/888 the use of tube pumps for the forwarding of liquids is known.

The drawings shows the construction of an embodiment of the invention.

The illustrated device for transferring an antiseptic solution comprises a tube 13 extending from a tank 11 containing the antiseptic solution to a nozzle 12 for spraying the solution, and a liquid feeder 14 for sending the solution from the tank 11 toward the nozzle 12 through the tube 13. In addition to the tube 13, a pressurized air supply tube 15 is connected to the nozzle 12. The tube 13 is made entirely of an elastic material such as vinyl chloride or silicone rubber and is 4 to 5 mm in outside diameter and about 1 mm in wall thickness. The liquid feeder 14 comprises a rectangular parallelepipedal main body 21, a tube case 22 mounted on the top of the main body and having a recessed portion 28, the recessed portion being circular when seen from above and having an open upper end, a cover 23 provided over the upper surface of the tube case, a main roller 24 disposed at the center of the recessed portion 28, and six press rollers 25 arranged on a circumference centered about the axis of rotation of the main roller 24 and surrounding the main roller 24. Although not shown, the main body 21 houses therein a gear mechanism for coupling the main roller 24 to the press rollers 25, a motor for driving the mechanism, etc. A start switch 26, a flow controller knob 27, etc. are mounted on the outer side of the main body 21. Formed between the peripheral surface of the recessed portion 28 and the press rollers 25 is a clearance which is not larger than the combined thickness of the lapping wall portions of the tube 13 produced when the tube 13 is collapsed flat to eliminate its hollow portion. Formed in the upper surface of the case 22 are two grooves 29 extending in parallel to each other tangentially of the circle of the peripheral surface of the recessed portion 28. A portion of the tube 13 is inserted in these grooves 29 and extends through the above-mentioned clearance. The press rollers 25 revolve around the main roller 24 while rotating each about its own axis. The peripheral speed of rotation of the press rollers 25 is equal to the peripheral speed thereof due to the revolution. Th direction of the rotation is opposite to that of the revolution. The speed is adjustable by the flow controller knob 27.

When the start switch 26 is turned on to drive the press rollers 25, the press rollers 25 move along the tube 13 while partially collapsing the tube 13, whereby the antiseptic solution is sent in a direction from the tank 11 toward the nozzle 12 through the tube 13.

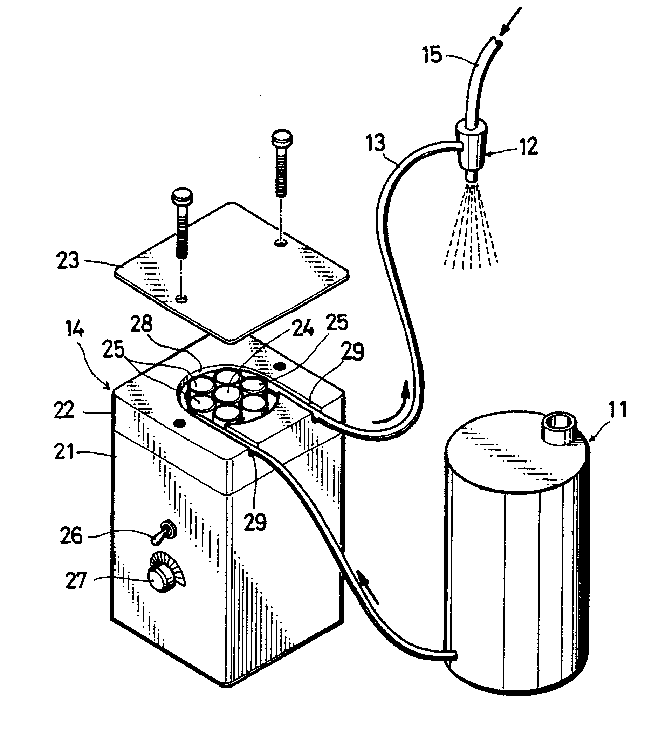

## Claims

1. A device for spraying a hydrogen peroxide water-solution on food packaging containers to be sterilized comprising : a tank (11) containing the hydrogen peroxide water solution, and a tube (13) for feeding the water-solution from the tank (11), to a spray nozzle (12) characterised in that

   a suction-type spraying nozzle (12) has been disposed at a front end portion of the water-solution feeding tube (13), and a pressurized air feeding means (15) for feeding pressurized air for spraying to the spraying nozzle, has been connected to the spray nozzle, whilst a tube pump (14), has been arranged in the vicinity of the water-solution feeding tube (13) for successively collapsing a part of the tube (13) made of elastic material over a required length from portion to portion in a direction from the tank (11) toward the nozzle (12) to thereby send the water-solution through the tube (13) in the same direction.

2. A device as defined in claim 1 wherein the tube pump (14) comprises:

   a tube case (22) formed in its upper surface with a recessed portion (28) having an open upper end and at least two tube insertion grooves (29), the recessed portion (28) having a peripheral surface of circular horizontal section, each of the tube insertion grooves (29) extending from a portion of the peripheral surface tangentially thereof, each tube insertion groove (29) having one end communicating with the interior of the recessed portion (28) and the other end left open at one side of the tube case (22),

   a plurality of press rollers (25) each having a vertical axis of rotation and arranged within the recessed portion (28) on a circumference concentric with the peripheral surface thereof, the press rollers (25) being spaced from the peripheral surface by a clearance not larger than the combined thickness of the lapping wall portions of the tube (13) produced when the part of the tube (13) made of the elastic material is collapsed flat to eliminate its hollow portion, and

   press roller drive means for revolving the press rollers (25) on the circumference and rotating the press rollers (25) each about its own axis at a peripheral speed equal to that of the press rollers (25) due to the revolution, in a direction opposite to the direction of the revolution.

## Revendications

1. Dispositif pour vaporiser une solution aqueuse à base de peroxyde d'hydrogène sur des récipients d'emballage d'aliments devant être stérilisés, comportant : un réservoir (11) contenant la solution aqueuse à base de peroxyde d'hydrogène et un tube (13) destiné à amener la solution aqueuse du réservoir (11) vers une buse de vaporisation (12), caractérisé en ce qu'une buse de vaporisation (12) du type à aspiration a été disposée au niveau d'une partie d'extrémité avant du tube (13) d'alimentation en solution aqueuse, et des moyens (15) d'alimentation en air sous pression destinés à amener vers la buse de vaporisation de l'air sous pression. destiné à la vaporisation ont été reliés à la buse de vaporisation tandis qu'une pompe à tube (14) a été prévue à proximité du tube (13) d'alimentation en solution aqueuse pour aplatir successivement une partie du tube (13), réalisé en un matériau élastique, sur une longueur requise, d'une portion à l'autre, dans une direction allant du réservoir (11) vers la buse (12) pour transmettre ainsi la solution aqueuse à travers le tube (13) dans la même direction.

2. Dispositif tel que défini dans la revendication 1, dans lequel la pompe à tube (14) comporte :

   une boîte à tube (22) pourvue, sur sa surface supérieure, d'une partie en creux (28) présentant une extrémité supérieure ouverte et au moins deux rainures (29) d'insertion du tube, la partie en creux (28) possédant une surface périphérique de section horizontale circulaire et chacune des rainures (29) d'insertion du tube s'étendant à partir d'une portion de la surface périphérique, tangentiellement par rapport à celle-ci, et ayant une première extrémité communiquant avec l' intérieur de la partie en creux (28) et la seconde extrémité ouverte au niveau de l'une des faces de la boîte à tube (22),

   plusieurs rouleaux presseurs (25) munis chacun d'un axe de rotation vertical et disposés à l'intérieur de la partie en creux (28) sur une circonférence concentrique par rapport à la surface périphérique de celle-ci, les rouleaux presseurs (25) étant séparés de la surface périphérique par un espace qui ne dépasse pas l'épaisseur combinée des portions superposées de paroi du tube (13) obtenue quand la partie du tube (13) réalisée en matériau élastique est aplatie pour éliminer sa portion creuse, et

   des moyens d'entraînement des rouleaux presseurs destinés a faire graviter les rouleaux presseurs (25) sur la circonférence et à faire tourner chacun d'eux autour de son propre axe à une vitesse périphérique égale à celle des rouleaux presseurs (25) en gravitation, dans une direction opposée à celle de la gravitation.

## Ansprüche

1. Eine Vorrichtung zum Versprühen einer Wasserstoffperoxid-Wasser-Lösung auf zu sterilisierende Lebensmittelverpackungsbehälter mit einem Tank (11), der eine Wasserstoffperoxid-Wasser-Lösung enthält, und einem Schlauch (13) für die Zuführung der Wasser-Lösung von dem Tank (11) zu einer Sprühdüse (12), dadurch gekennzeichnet, daß an einem vorderen Endabschnitt des Wasserlösungszuführschlauches (13) eine Saugtyp-Sprühdüse (12) angeordnet ist und daß mit der Sprühdüse ein Druckluftzuführmittel (15) zur Zuführung von Druckluft zu der Sprühdüse zum Zwecke des Versprühens verbunden ist, während eine Schlauchpumpe (14) zum sukzessiven Zusammendrücken eines Teils des aus elastischem Material hergestellten Schlauches (13) über eine benötigte Länge abschnittsweise in einer Richtung vom Tank (11) zu der Düse (12) in der Nähe des Wasserlösungszuführschlauches (13) angeordnet ist, um hierdurch die Wasser-Lösung durch den Schlauch (13) in die gleiche Richtung zu befördern.

2. Eine Vorrichtung nach Anspruch (1), wobei die Schlauchpumpe (14) ein Schlauchgehäuse, (22) aufweist in deren Oberfläche ein vertiefter Abschnitt (28) mit einem oben offenen Ende und zumindest zwei Schlaucheinsatznuten (29) eingeformt ist, wobei der vertiefte Abschnitt (28) eine Umfangsoberfläche mit kreisförmigem Horizontalquerschnitt hat und jeder der Schlaucheinsatznuten (29) sich tangential von einem Abschnitt der Umfangsoberfläche erstreckt und jede Schlaucheinsatznut (29) ein Ende hat, das mit dem Innenraum des vertieften Abschnittes (28) Verbindung hat, und ein anderes Ende, das auf einer Seite des Schlauchgehäuses (22) offen ist, eine Vielzahl von Druckrollen (25), die jeder eine vertikale Drehachse haben und innerhalb des vertieften Abschnittes (28) auf einer Kreislinie angeordnet sind, die konzentrisch mit der Umfangsoberfläche ist, wobei die Druckrollen (25) durch einen Freiraum einen Abstand zu der Umfangsoberfläche haben, der nicht größer ist als die Gesamtdicke der sich überlappenden Wandungsabschnitte des Schlauches (13), welche entsteht, wenn der aus elastischem Material bestehende Teil des Schlauches (13) flach zusammengedrückt ist, um seinen Hohlraum zu eliminieren, sowie Druckrollenantriebsmittel aufweist zum Drehen der Druckrollen (25) auf der Kreislinie und zum Rotieren der Druckrollen (25) jeder um ihre eigene Achse in einer Richtung entgegengesetzt der Richtung der Drehung mit einer Umfangsgeschwindigkeit, die gleich ist derjenigen der Druckrollen (25) durch die Drehung.